# EUROPEAN PATENT APPLICATION

(11) **EP 4 230 172 A1**
(43) Date of publication of application: **23.08.2023**
(21) Application number: 22315033.5
(22) Date of filing: 17.02.2022
(51) Int. Cl.: A61B 34/20, A61B 34/00, A61B 34/30, A61B 90/00

(54) **SURGICAL ROBOTIC SYSTEM FOR CEMENTOPLASTY**

(71) Applicant: Ecential Robotics, 38610 Gieres (FR)
(72) Inventor: DAUNE, Gautier, 38610 Gières (FR); BEREAU, Marc, 38610 Gières (FR)
(74) Representative: INNOV-GROUP

(57) **Abstract**

A surgical robotic system (1) comprising a robotic arm (4), an injection needle supported by the robotic arm (4), a localization system (5), an imaging system (3), at least one control unit (14, 15, 16). At least one control unit (14, 15, 16) is configured to perform at least one controlled loop carried out after a dispense of cement with the injection needle, in the region of interest of a patient. This controlled loop comprises
- a step of imaging the region of interest, with the imaging system (3), for providing an updated set of imaging data,
- a step of using at least partially the updated set of imaging data, for calculating an updated set of injection parameters,
- a step of using at least one parameter of the updated set of injection parameter for controlling the dispense of cement through the injection needle.

A method for controlling a surgical robotic system (1).

## Description

### Field of invention

The invention relates to a surgical robotic system for cementoplasty and a method for controlling such a system.

### Technical background

Cementoplasty refers to percutaneous or open surgery injections of cement in or on a bone of a patient anatomy. For example, cementoplasty is used to stabilize the spine, the skeletal system, or any bone of the human body, to treat or prevent vertebral and extraspinal pathological fractures and to relieve pain in patients with osteoporosis and bone metastases. Cementoplasty term is broadly used to include surgical procedures such as vertebroplasty, kyphoplasty, osteoplasty, sacroplasty, etc..

Nowadays, such surgical procedures involve the use of X-ray imaging as a safety check during the procedure in order to avoid possible problems such as cement leakage due to the presence of failure(s) on the wall of the cavity to be filled or the non-uniformity of the cavity filling.

After each cement injection the operator (e.g., a surgeon) has to produce a two-dimensional (2D) shoot of the region of interest with an X-ray imaging system to check if the cement has been properly dispensed. One of the main disadvantages of such procedures is the risk of X-ray exposition of the operator during this check.

One of the most efficient ways to reduce the radiation exposure of operators during such procedures is to assist the operators with a surgical robotic system, e.g., a surgical system comprising a robotic arm.

A robot-based method to assist an operator is disclosed for example in the patent document US20190254750A1. This method for controlling the dispensing of a cement contained in an injection needle actuated by a robotic arm, comprises
a) a step of dispensing a volume of cement through the tip of the injection needle moved by a robotic arm, and
b) a step of imaging the bone and the quantity of cement dispensed at the step a).

An aim of the invention is to improve the control of the cement dispensing.

### Summary

According to a solution, a surgical robotic system according to claim 1 is disclosed.

Indeed, the system according to the invention allows for updating as it goes along, thanks to at least the imaging system, at least one of the parameters used for controlling the cement injection. The controlled loop provides a security check, as well as a better control of the cement dispensing. For example, a withdrawal trajectory of the injection needle may be more accurately defined than with what prior art systems and methods can provide. One can note that "controlling the dispense of cement in the region of interest through the injection needle" includes stopping the dispense of cement in the region of interest through the injection needle, if at least one updated parameter triggers such an operation.

More particularly, the cement injection may be based on a set of parameters the values of which are defined according to data obtained from the surgical robotic system according to the invention. For example, these parameters are comprised in the following list:
- the insertion trajectory,
- the withdrawal trajectory,
- the volume of cement already dispensed,
- the volume of a cavity which remains to be filled with the cement,
- the location and/or the shape of the cement already dispensed,
- the location of the tip of the injection needle,
- the distance between the tip and the cement already dispensed,
- the appearance of a leak of cement,
- etc.

For example, there is an advantage in inserting the tip of the injection needle as close as possible to where the cement is to be optimally deposited. However, it may be necessary to prevent the injection needle from getting stuck in the cement. Thanks to the invention, at least one injection parameters can be precisely defined so as to prevent such a problem.

The system according to the invention may also optionally include one and / or the other of the features of any one of claims 2 to 4.

The disclosure also relates to a method according to any one of claims 5 to 14, as well as a computer program according to claim 15.

### Brief description of the drawings

Other features, objects and advantages of the invention will become apparent from reading the detailed description that follows, and the attached drawings, given as non-limiting examples and in which:
FIG. 1 schematically shows in perspective an example of implementation of a surgical robotic system according to the invention;
FIG. 2 schematically shows in perspective the robotic arm of the surgical robotic system shown in FIG. 1;
FIG 3 schematically represents successive injection cycles performed according to an example of method according to the invention;

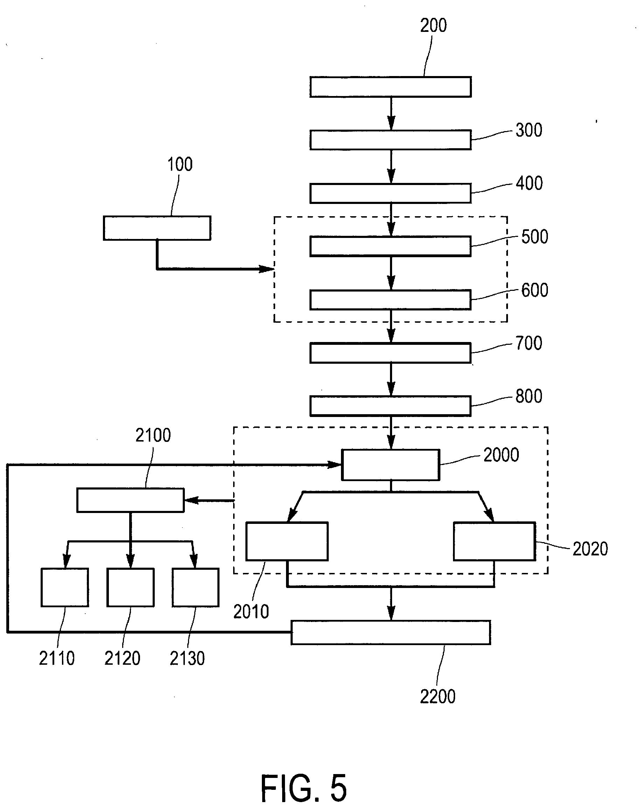

FIGs. 4 and 5 correspond to diagrams representing respectively an example of autonomous mode and an example of semi-autonomous mode of implementation of the method according to the invention.

### Detailed description

An example of embodiment of a surgical robotic system is described below.

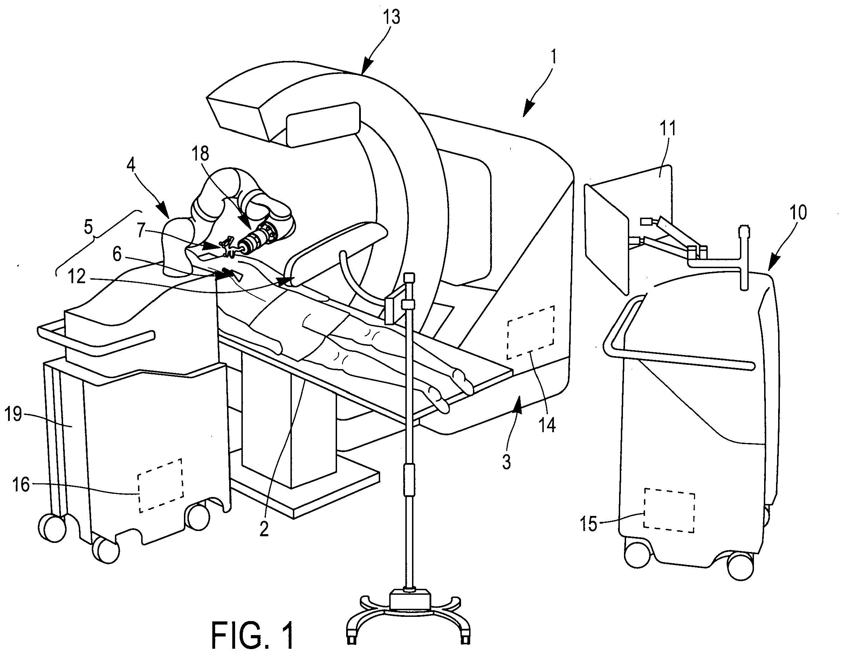

FIG. 1 illustrates an embodiment of a surgical robotic system 1 in an operating room, in a context of spine surgery. The operating room comprises an operating table 2, an imaging system 3, a robotic arm 4 and a localization system 5. For spine surgery, the patient may be in prone position on the operating table 2.

### Localization system

The localization system 5 may comprise a camera 12 and trackers 6, 7 (See FIGs. 1, 2 and 3). In particular these trackers may comprise at least one patient tracker 6 and at least one robot tracker 7.

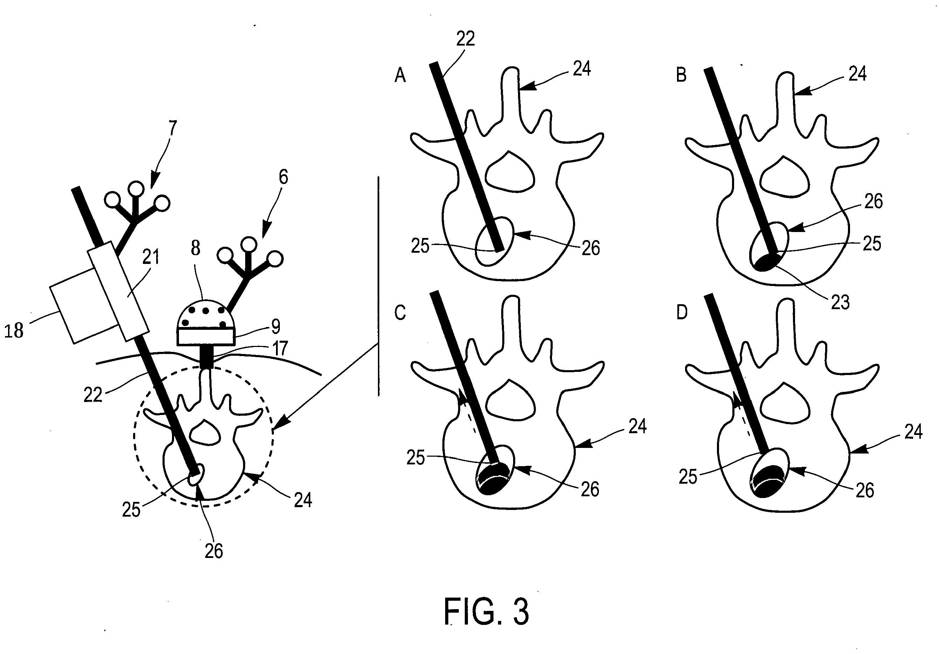

For example, a patient reference 9 is rigidly fixed on the patient body (e.g. as described in EP3361957), in particular to a vertebra in the case of spine surgery. For example, the patient reference 9 is maintained on the patient body with pins 17 sank into a bone (e.g. a vertebra) of the patient (see FIG.3). The patient tracker 6 may be then attached to the patient reference 9.

For example, the robot tracker 7 is attached to an end-effector 18, or to a tool guide or tool holder 21, located at the distal end of the robotic arm 4. The localization system 5 may be already calibrated or calibrated during surgery, said calibration may comprise a definition of the spatial relationship between the robot tracker 7 and the tool guide or tool holder 21 in which the injection needle 22 is positioned.

The localization system 5 may be chosen among various technologies, such as optical localization, electromagnetic localization, or ultrasound localization. In the illustrated embodiment, the localization system 5 comprises an infra-red camera 12 arranged to detect optical trackers 6, 7, wherein each optical tracker 6 or 7 comprises a set of reflective markers, having for example a spherical shape.

The localization system 5 is configured and calibrated for providing data relating to the position of the patient tracker 6 (which allows knowing the position of the patient reference 9), relative to the robot tracker 7 (which allows knowing where a tool rigidly linked to the robotic arm 4 is).

### Imaging system

The imaging system 3 may comprise an X-ray imaging system such as a C-arm 13 (e.g., as described in EP2868277A1 or EP3359042A1) or a CT scan. The imaging system 3 may comprise its own control unit 14 with at last one processor, at least one data storage device (communication means such as at least one of the following means: communication ports, display screen, keyboard, etc. may be connected to the control unit 14). The whole control unit 14 or part of it may be integrated in the X-ray imaging system itself, or it may be placed in or on a cart or it may be remotely placed. The control unit 14 of the imaging system 3 controls motors which move the C-arm 13 with regard to the patient. Each motor is associated to an encoder that provides at any time the relative position of the X-ray imaging system with respect to a reference position. When a 2D X-ray image is acquired, the corresponding position of the imaging system is recorded. Thus, each 2D image is recorded in the referential of the imaging system.

The imaging system 3 acquires and provides intraoperative 2D images of the patient. These 2D images are processed according to a reconstructing three-dimensional (3D) method (e.g. as described in EP3175790A1).

In particular, as explained in EP3175790A1, a calibration phantom 8 may be used. The phantom 8 may comprise radiopaque fiducials having a known shape and size (e.g. balls or pins) arranged in known position and which form a particular pattern which is visible on each 2D image, so as to allow for a registering of X-ray images relative to each other. The projection on each 2D image of the known 3D geometry of the radiopaque fiducials of the phantom 8, allows reconstructing 3D images and more particularly the 3D images of the region of interest (with, for example a cavity 26 in a bone - see Fig. 3)

### Robotic arm

The robotic arm 4 may comprise a control unit 16 for controlling the movements of the robotic arm 4. An example of robotic arm 4 is described in EP3821843A1. The imaging system 3 and the robotic arm 4 may provide navigation capabilities when coupled to the localization system 5 (e.g. as described in EP3361977A1). The robotic arm 4 may be supported by a mobile cart 19, which is immobilized at a fixed location relative to the operating table 2 during the surgical intervention.

The respective control units 14, 16 of the imaging system 3 and the robotic arm 4, as well as a control unit 15 of a core station 10 may belong to a unified control system. The core station 10 may comprise at least one display screen and computer power means. The core station 10 may run a master computer-readable program. A "unified control system" means in the present text that one of said control units 14, 15, 16 runs a master computer-readable program and that at least one of the other control units runs a slave computer-readable program, the master program being configured to send commands to the slave program(s) and to receive data (e.g. status) from the slave program(s). For example, the control unit 16 of the robotic arm 4 may run a master program to control movements of the robotic arm 4, while the control unit 14 of the imaging system 3 runs a slave program to control movements of the motorized C-arm 13 and/or acquire X-ray images. Conversely, the control unit 14 of the imaging system 3 may run a master program to control movements of the motorized C-arm 13 and/or acquire X-ray images, while the control unit 16 of the robotic arm 4 runs a slave program. In other embodiments, the master program can be executed by the control unit of another system (e.g. the control unit 15 of the core station 10) and the control units 14, 16 of the robotic arm 4 and the imaging system 3 can both run slave programs. Thanks to such a unified control system, it is possible to synchronize actions of the robotic arm 4 and of the imaging system 3, since each system has knowledge of the position and status of the other system.

The unified control system advantageously comprises a user interface (e.g. display screens 11 on the core station 10) adapted to display information to a user.

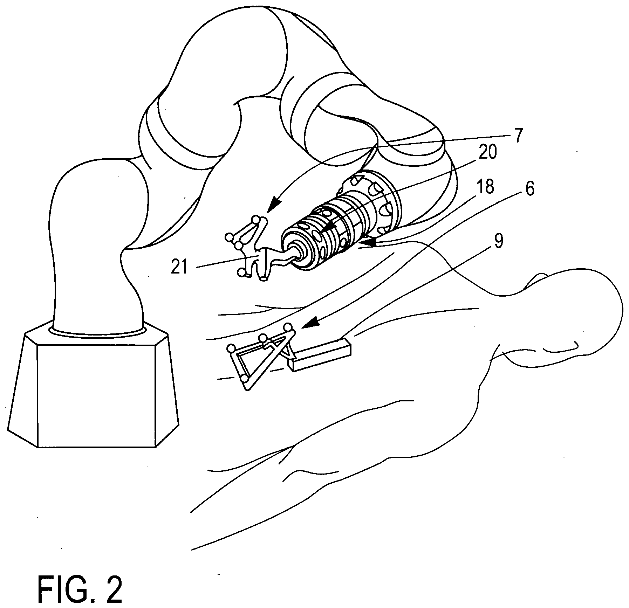

The end effector 18 is described in greater detail with reference to FIG. 2. The end effector 18 comprises control buttons 20. The robot tracker 7 and a tool guide or tool holder 21 are mounted on the end effector 18 (see FIGs. 2 and 3). The control buttons 20 may be used in particular for hand-guiding (allowing the user to move the robot arm as she/he wishes). The control buttons may also be used for triggering the movement of the robotic arm 4 to a target position and/or in a target configuration. The tool guide or tool holder 21 may be configured for actuating an injection needle 22 (see FIG. 3). The injection needle 22 is adapted for dispensing a cement 23 (see FIG. 3). The cement 23 is advantageously radiopaque (e.g., it comprises fluorescent particles).

The control unit 16 of the robotic arm 4 may be accommodated in the cart 19. Alternatively, the control unit 16 may be separated and at a distance from the robotic arm 4. This control unit 16 may also control at least partly the movements of the cart 19. This control unit 16 comprises at last one processor, at least one data storage device (communication means such as at least one of the following means: communication ports, display screen, keyboard, etc. may also be connected to the control unit 16).

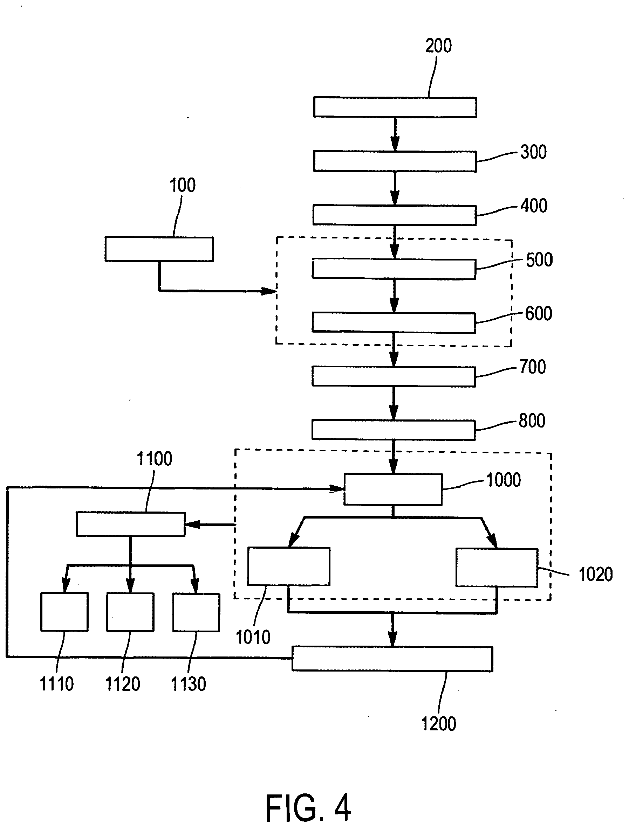

An example of implementation of a method according to the invention is described below in connection with FIG. 4.

A pre-operative planning step 100 may be implemented for defining an initial trajectory for the injection needle 22. The initial trajectory may be computed so as to be optimal. The term "trajectory" in this document may include in particular one or several angles of an axis (e.g. the longitudinal axis) of the injection needle 22 with regard to the patient body or a region of interest or a target in the patient anatomy (e.g. a bone such as a vertebra 24 - see FIG. 3), and one or several locations or positions of the tip 25 of the injection needle 22 in the region of interest, during the movement of the injection needle 22 toward the region of interest and/or into the region of interest and/or from the region of interest. The pre-operative planning step 100 may be based on the acquisition and processing of 2D and/or 3D images by the imaging system 3. The pre-operative planning step 100 may also take into account data obtained from other apparatuses than the imaging system 3 (even from a completely different imaging system for example). The pre-operative planning step 100 may also comprise determining at least one volume of a region of interest (e.g., a cavity 26 in a bone), at least one quantity (e.g., a weight or a volume) and/or at least one flow rate, of cement to be dispensed in such a region of interest. A total quantity of cement to be dispensed in the region of interest may be defined at the pre-operative planning step 100. The pre-operative planning step 100 may comprise then computing an initial position, or several successive initial positions (e.g. a trajectory), of the tip 25 of the injection needle 22 during the dispense of cement 23, as well as possibly an initial angle, or several successive initial angles, of the injection needle 22 relative to the region of interest.

As an alternative to the calculation of an initial trajectory, based on 2D and/or 3D images, for the injection needle 22, at the pre-operative planning step 100, it may also be possible to define targets directly in the coordinate system of the patient reference 9 attached to the patient. Such targets may be used for guiding an operator when moving the robotic arm 4 and/or the injection needle 22, or for automatically moving the robotic arm 4.

Furthermore, the patient is prepared for the cementoplasty. To do so, the patient is placed on the operating table 2. The patient reference 9 is rigidly fixed on the patient body. The phantom 8 is rigidly attached onto the patient reference 9. The operators can then move to a location where they are not exposed to X-rays when the C-arm 13 is on and generates X-rays.

Then step 200 is implemented. The imaging system 3 acquires series of 2D images which will be used at step 300 for a 3D reconstruction. This step 200 allows in particular to see on the X-ray images the pins 17, and the radiopaque fiducials of the phantom 8. The radiopaque fiducials of the phantom 8 having a known shape and size (e.g. balls or pins) arranged in known position, it allows registering relative to each other the 2D images acquired by the imaging system 3.

At step 300, at least one 3D image of at least one region of interest in the patient anatomy is reconstructed based on the 2D X-ray images obtained from step 200. As already mentioned, radiopaque fiducials of the phantom 8 having a known shape and size (e.g. balls or pins) arranged in known position, it allows registering the 2D images in particular in view of the 3D reconstruction.

The patient tracker 6 is rigidly attached to the patient reference 9. The phantom 8 can be removed from the patient reference 9.

Both the patient tracker 6 and the robot tracker 7 comprise reflective markers which can be seen by the infrared camera 12. The patient tracker 6 having a precisely known position on the patient reference 9, and its reflective markers having a precisely known geometry and position on the patient tracker 6, the correlation can be made between the X-ray images and the infrared images.

Further, the robot tracker 7 has a known geometry and the tool, i.e., the injection needle 22, is rigidly attached to the tool guide or tool holder 21 which is itself rigidly linked to the robot tracker 7. The injection needle 22 has a known geometry. Therefore, the position of the robot tracker 7 being precisely tracked by the infrared camera 12, the position of the injection needle 22 (in particular the angle of an axis of the injection needle 22 relative to the region of interest and the position of its tip 25 relative to the region of interest) is determined thanks to the calibration of the localization system 5 and displayed on a display screen for example as a design representing the injection needle 22 in the 3D image reconstructed from the 2D X-ray images. An operator is then able to see on the 3D reconstructed images the profile of the tracked instrument (e.g. the injection needle 22 and its tip 25) and/or the axis of the tracked instrument, when this instrument has penetrated in the patient body. The operator will then be able to track how and where the injection needle 22 and the tip 25 are moved in the patient body.

At step 400, the region of interest where the cement has to be dispensed is defined and/or monitored, based at least on the 2D and/or 3D images obtained from steps 200 and/or 300. For example, said region of interest can be segmented using image segmentation algorithm like edge detection, dual clustering method, region-growing methods or clustering methods (especially agglomerative clustering, spectral clustering, density-based spatial clustering of applications with noise (DBScan)).

At step 500, an initial position of the tip 25 of the injection needle 22 in the region of interest is defined. This initial position (possibly along an optimal trajectory) of the tip 25 may have been calculated at step 100. Possibly, this initial position is defined according to the pre-operative data obtained from step 100 (possibly, as explained above, with data obtained from other apparatuses) and/or per-operative data obtained from at least one of the steps 200 to 400. This initial position of the tip 25 corresponds to the position where the tip 25 will be located for performing the first cement injection in the region of interest. The initial position of the tip 25 is comprised in an initial set of injection parameters, said initial set of injection parameters being defined either during pre-operative planning step 100 and/or during one of the per-operative steps 200 to 600. The initial set of injection parameters comprises at least one of the parameters of the list comprising an insertion trajectory, a withdrawal trajectory (by default the reverse of the insertion trajectory), a location where the tip of the injection needle is expected to be placed in the region of interest, a speed of withdrawal of the tip of the injection needle from the region of interest, a volume of cement to be dispensed in the region of interest, and an injection flow rate for the cement to be injected through the tip of the injection needle in the region of interest. This step 500 may also take into account data related to the environment configuration during the surgical operations. In particular, the environment configuration is determined by other elements and/or apparatuses present in the operating room (e.g. the C-arm 13) and the robotic arm configuration is caused both by the positioning of the tool guide or tool holder 21, in view of guiding the injection needle 22 before and during its insertion in the region of interest, and by the environment configuration.

At step 600 the initial trajectory for the insertion, or the withdrawal, of the injection needle 22 in, or from, the region of interest is computed if it has not been done yet, or otherwise checked and possibly modified. This step 600 uses data derived from the pre-operative planning step 100 and/or data derived from at least one of the per-operative steps 200 to 500. The initial trajectory comprises the initial position of the tip 25, and possibly one or several other positions that the tip 25 should occupy during the insertion and/or the withdrawal of the injection needle 22 in, or from, the region of interest. The angle, position, insertion trajectory, etc. of the injection needle 22 and its tip 25 for the insertion in the patient body, are known now. The insertion of the injection needle 22 in the patient body can be performed.

Then, at step 700, the robotic arm 4, supporting or not the injection needle 22, is automatically placed, at the position and with the angle previously computed as being appropriate either for positioning the injection needle 22 in the vicinity of the patient body or for inserting the injection needle 22 in the patient body. If the robotic arm 4 does not support the injection needle 22, the robotic arm 4 is automatically placed at the position and with an angle which is appropriate for subsequently positioning the injection needle 22 in the tool guide or tool holder 21. Alternatively, the operator (e.g. a surgeon) handles herself/himself the robotic arm 4 so as to place it at the position and with the angle previously computed as being appropriate either for positioning the injection needle 22 in the vicinity of the patient body or for inserting the injection needle 22 in the patient body. In any case, the positioning of the robotic arm 4 can be monitored thanks to the localization system 5.

Then, the operator positions the injection needle 22 on the robotic arm 4, in the tool guide or tool holder 21, if not already done.

At step 800, the operator, or the robotic arm 4 itself if it operates in fully autonomous mode, inserts the injection needle 22 in the region of interest, according to the position and angle constrained by the robotic arm 4, and places the tip 25 of the injection needle 22 at, or close to, the optimal position which is determined by the surgical robotic system 1. This position may correspond to the initial position computed at step 100 and/or 500, or it may correspond to a position chosen by the operator according, for example, to what she/he sees on the 3D reconstructed images.

Then the method according to the invention may differ as a function of the autonomy left to the robotic arm 4.

Two different levels of autonomy of the robotic arm 4 will be described. A level corresponding to relatively high level of autonomy of the robotic arm 4 and a relatively low level of autonomy of the robotic arm 4. But, of course, other variations of the method according to the invention may be implemented with other levels of autonomy of the robotic arm 4 (e.g., mixing the variations described below).

### Autonomous mode:

At step 1000, a volume of cement 23 is dispensed in the region of interest (e.g., a vertebra cavity 26). This injection is controlled by the surgical robotic system 1. Such a control of the injection by the surgical robotic system 1 may involve an activation of the injection performed by another device (i.e. injection activation means) which is coupled or not to the robotic arm 4.

More particularly, this step 1000 may be performed according to several variations.

According to a first variation, at step 1010, the cement 23 is dispensed at a flow rate which has been previously defined. For example, the flow rate was defined as one of the parameters of the initial set of injection parameters (see step 500 for example). The flow rate corresponds to a volume of cement 23 which is dispensed during a period of time. Therefore, the robotic arm 4 may allow to control for example, the flow rate and the time of dispense of the cement 23. These flow rate and time may be comprised in the initial set of injection parameters. This first variation has the advantage of providing a more autonomous mode.

According to a second variation, at step 1020, the cement is dispensed at a flow rate which is adapted considering data derived from images resulting from the previous injection cycle(s) (or from the initial set of injection parameters for the first cement injection). This second variation has the advantage of providing more optimisation.

After each dispense of a volume of cement 23, the tip 25 of the injection needle 22 may be moved along a withdrawal trajectory, during a step 1100 of withdrawal. Alternatively, the tip 25 of the injection needle 22 remains at the same location until the total volume of cement sufficiently fills the cavity 26. Alternatively, the tip 25 of the injection needle 22 is moved after a variable number of injections.

If the tip 25 of the injection needle 22 is moved along a withdrawal trajectory, the method according to the invention may comprise a withdrawal step 1110 which is performed continuously along a dispense cycle, or it may comprise a withdrawal step 1120 which is discreetly performed along a dispense cycle, or it may comprise a withdrawal step 1130 only at the end of each dispense of cement 23 (i.e., at the end of each cycle - See FIG. 4).

For each one of these cases, the withdrawal steps 1110, 1120, 1130 are controlled and managed, for example via master to slave instructions, by at least one of the control units 14, 15, 16, respectively the imaging system 3, the robotic arm 4 and the core station 10, based in particular to data provided by the localization system 5 and the imaging system 3.

In any case, advantageously, a step 1200 of safety or control capture of 2D X-ray images is performed before the next dispense(s) of cement 23. Safety or control X-ray images may be used for optimizing the injection parameters for the next injection cycles.

Therefore, steps 1000 to 1200 are comprised in a controlled loop which is repeated as long as the volume of dispensed cement 23 is not considered as sufficient by an operator, and/or as long as the cavity 26 is not filled, and/or as long as the volume contained in the injection needle 22 is not completely dispensed, and/or as long as a predetermined volume is not completely dispensed, etc.

In FIG. 3, four schematical images A, B, C, D are represented. For example, image A corresponds to the image seen by the operator when she/he initially positions the tip 25 of the injection needle 22. Alternatively, according to another example, only images B, C, D are displayed. Indeed, checking the position of the tip 25 and the injection angle is not always necessary, in particular since it may be known from data provided by the localization system 5. In this case, the safety or control X-ray images are acquired and displayed only after a cement injection cycle. Each one of the images B, C, D corresponds, for example, to an image of the region of interest as captured at step 1200 of successive injection cycles. Successively a volume of cement 23 is dispensed, the tip 25 is placed at a new location, a volume of cement 23 is dispensed, the tip 25 is placed at a new location, etc. During this discrete process, the total volume of cement already dispensed may be monitored with the imaging system 3 so as to be able for example to detect a possible leak of cement. During this discrete process, the withdrawal trajectory of the tip 25 may be adapted by at least one of the control units 14, 15, 16, of respectively the imaging system 3, the robotic arm 4 and the core station 10, according to data provided by the localization system 5 and the imaging system 3, and possible master/slave instructions. The imaging system 3 captures 2D X-ray images of the region of interest. These images are used for providing the information needed by the robotic arm 4 for positioning, for the next cycle, the tip 25 of the injection needle 22 (e.g., through master and slave programs of a unified system as mentioned above).

### Semi-autonomous mode:

Steps 100 to 800 are identical or similar to the ones already described in connection with the autonomous mode.

At step 2000, a volume of cement 23 is dispensed in the region of interest (e.g., a vertebra cavity 26). This injection is controlled by the operator.

More particularly, this step 2000 may be performed according to several variations.

According to a first variation, at step 2010, the cement 23 is dispensed by the operator at a flow rate which has been previously defined. For example, the flow rate was defined as one of the parameters of the initial set of injection parameters (see step 500 for example). The operator may control the flow rate thanks to information provided by a flow rate sensor.

According to a second variation, at step 2020, the cement is dispensed at a flow rate which is adapted considering data derived from images resulting from the previous injection cycle(s) (or from the initial set of injection parameters for the first cement injection). The operator may control the adapted flow rate thanks to information provided by a flow rate sensor.

Basically, the respective advantages of each one of these variations are similar to the ones already mentioned above in connection with the autonomous mode.

After each dispense of a volume of cement 23, the tip 25 of the injection needle 22 is moved along a withdrawal trajectory, during a step 2100 of withdrawal. Alternatively, the tip 25 of the injection needle 22 remains at the same location until the total volume of cement sufficiently fills the cavity 26. Alternatively, the tip 25 of the injection needle 22 is moved after a fixed or variable number of injections.

If the tip 25 of the injection needle 22 is moved along a withdrawal trajectory, the method according to the invention may comprise a withdrawal step 2110 which is performed continuously along a dispense cycle, or it may comprise a withdrawal step 2120 which is discreetly performed along a dispense cycle, or it may comprise a withdrawal step 2130 only at the end of each dispense of cement 23 (i.e., at the end of each cycle).

For each one of these cases, the withdrawal steps 2110, 2120, 2130 are controlled and managed by the operator, who can be helped for example by what she/he sees on 2D and/or 3D images. The operator is helped or guided by the information and data computed, updated and provided by the surgical robotic system 1.

In any case, advantageously, a step 2200 of safety or control capture of 2D X-ray images is performed before the next dispense(s) of cement 23. Safety or control X-ray images may be used for optimizing the injection parameters for the next injection cycles.

Therefore, steps 2000 to 2200 are comprised in a controlled loop which is repeated as long as the volume of dispensed cement 23 is not considered as sufficient by an operator, and/or as long as the cavity 26 is not filled, and/or as long as the volume contained in the injection needle 22 is not completely dispensed, and/or as long as a predetermined volume is not completely dispensed, etc..

## Claims

1. A surgical robotic system (1) comprising
- a robotic arm (4),
- an injection needle (22) with a tip (25) having an orifice configured for dispensing a quantity of cement (23) contained in the injection needle (22), the injection needle (22) being supported by the robotic arm (4),
- a localization system (5) configured for providing data relating to the position of a patient reference configured for being rigidly fixed to a patient body, relative to a robot tracker (7) supported by the robotic arm (4) and whose spatial relationship with the injection needle (22) is known,
- an imaging system (3) and
- at least one control unit (14, 15, 16),
**characterized in that**
said at least one control unit (14, 15, 16) is configured to perform at least one controlled loop comprising at least
- a step of imaging the region of interest with the imaging system (3), this step of imaging providing an updated set of imaging data,
- a step of using at least partially the updated set of imaging data, for calculating an updated set of injection parameters,
- a step of using at least one parameter of the updated set of injection parameter for controlling the dispense of cement (23) in the region of interest through the injection needle (22).

2. A surgical robotic system (1) according to claim 1, wherein the updated set of injection parameters comprises at least one of the parameters of the list comprising a withdrawal trajectory, a location of the tip (25) of the injection needle (22) in the region of interest for a subsequent dispense of cement (23), a volume of cement (23) to be dispensed in the region of interest before carrying out a subsequent controlled loop, an injection flow rate of cement (23) to be injected through the tip (25) of the injection needle (22) in the region of interest, a configuration of the robotic arm (4) for supporting the injection needle (22) during the subsequent dispense of a volume of cement (23), a configuration of the robotic arm (4) for supporting the injection needle (22) during the subsequent step of imaging, a distance between the tip (25) of the injection needle (22) and the cement already dispensed in the region of interest.

3. A surgical robotic system (1) according to claim 1 or 2, comprising injection activation means for controlling the dispense of cement contained in the injection needle (22), in the region of interest.

4. A surgical robotic system (1) according to claim 3, wherein the injection activation means for controlling the dispense of cement contained in the injection needle (22) are themselves controlled by the said at least one control unit (14, 15, 16).

5. A method for controlling a surgical robotic system (1) comprising a robotic arm (4), a localization system (5) and an imaging system (3), the surgical robotic system (1) being configured for assisting an operator in dispensing, in a region of interest comprising a bone of a patient anatomy, a cement through a tip (25) of an injection needle (22), and the localization system (5) being configured and calibrated for providing data relating to the position of a patient reference configured for being rigidly fixed to a patient body, relative to a robot tracker (7) supported by the robotic arm (4) and rigidly linked to the injection needle (22), the method comprising
- a step of imaging the region of interest, with the imaging system (3), this step of imaging providing an initial set of imaging data,
- a step of calculating an initial set of injection parameters, at least partially based on the initial set of imaging data,
- a step of positioning the tip (25) of the injection needle (22), at a location defined in using at least partially the initial set of imaging data, and data provided by the localization system (5),
the method further comprising
at least one controlled loop comprising at least
- a step of dispensing, through the tip (25) of the injection needle (22), a quantity of cement (23) in the region of interest;
- a step of imaging the region of interest, with the imaging system (3), this step of imaging providing an updated set of imaging data,
- a step of using at least partially the updated set of imaging data, for calculating an updated set of injection parameters,
- a step of using at least one parameter of the updated set of injection parameters for controlling the dispense of cement (23) in the region of interest through the injection needle (22).

6. The method of claim 5, wherein said initial set of parameters comprises at least one of the parameters of the list comprising an insertion trajectory, a withdrawal trajectory, a location where the tip of the injection needle is expected to be placed in the region of interest, a speed of withdrawal of the tip of the injection needle from the region of interest, a volume of cement to be dispensed in the region of interest, and an injection flow rate for the cement to be injected through the tip of the injection needle in the region of interest.

7. The method of claim 6, comprising a step of using data provided by the localization system (5) for positioning the tip (25) of the injection needle (22), at a location of the updated withdrawal trajectory.

8. The method according to any one of claims 5 to 7, wherein during said step of dispensing, the quantity of cement (23) dispensed in the region of interest is controlled by the surgical robotic system (1).

9. The method according to any one of claims 5 to 8, wherein during said step of dispensing, the quantity of cement (23) dispensed in the region of interest is controlled by an operator.

10. The method according to any one of claims 5 to 9, wherein the step of positioning the tip (25) of the injection needle (22) at a location of a withdrawal trajectory is controlled by the surgical robotic system (1).

11. The method according to any one of claims 5 to 10, wherein the step of positioning the tip (25) of the injection needle (22) at a location of a withdrawal trajectory is performed at the end of each step of dispensing a quantity of cement (23).

12. The method according to any one of claims 5 to 10, wherein the step of positioning the tip (25) of the injection needle (22) at a location of a withdrawal trajectory is performed continuously during the step of dispensing a quantity of cement (23).

13. The method according to any one of claims 5 to 10, wherein the step of positioning the tip (25) of the injection needle (22) at a location of a withdrawal trajectory is performed discreetly during the step of dispensing a quantity of cement (23).

14. The method according to any one of claims 5 to 13, wherein the withdrawal trajectory is determined so as to keep tip (25) of the injection needle (22) outside the injected cement (23).

15. A computer program comprising instructions which, when the program is executed by a computer, cause the computer to carry out the method according to any of claims 5 to 14.
